(19) Europäisches Patentamt
European Patent Office
Office européen des brevets

(11) Veröffentlichungsnummer: **0 415 880 B1**

# (12) EUROPÄISCHE PATENTSCHRIFT

(45) Veröffentlichungstag der Patentschrift: **05.04.95**

(51) Int. Cl.[6]: **C09D 11/00**, C08K 5/3475, C08K 5/42, C07D 249/20, D21H 17/05

(21) Anmeldenummer: **90810617.2**

(22) Anmeldetag: **16.08.90**

## (54) Lichtstabilisierte Tinten.

(30) Priorität: **25.08.89 CH 3085/89**
**17.01.90 CH 141/90**

(43) Veröffentlichungstag der Anmeldung:
**06.03.91 Patentblatt 91/10**

(45) Bekanntmachung des Hinweises auf die Patenterteilung:
**05.04.95 Patentblatt 95/14**

(84) Benannte Vertragsstaaten:
**DE FR GB IT**

(56) Entgegenhaltungen:
**EP-A- 0 147 747**
**EP-A- 0 374 751**
**FR-A- 2 252 341**
**FR-A- 2 619 814**

**DATABASE WPIL, no. 82-4917E, Derwent Publications Ltd, London, GB; & JP-A-57 074 193**

(73) Patentinhaber: **CIBA-GEIGY AG**
**Klybeckstrasse 141**
**CH-4002 Basel (CH)**

(72) Erfinder: **Laver, Hugh Stephen, Dr.**
**Route de Schiffenen 12**
**CH-1700 Fribourg (CH)**

EP 0 415 880 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft wässrige Tinten, die als Lichtschutzmittel ein wasserlösliches Derivat eines 2-(2'-Hydroxyphenyl)-benztriazols enthalten. Diese Tinten sind vor allem für den Tintenstrahldruck geeignet. Ferner betrifft die Erfindung neue sulfonierte 2-(2'-Hydroxyphenyl)-benztriazole.

Das Drucken mittels Tintenstrahl ist ein sehr schnelles Druckverfahren, das durch elektrische Signale gesteuert wird. Dabei wird ein feiner Strahl von Tintentröpfchen durch eine Düse auf das Aufzeichnungsmaterial gespritzt. Als Tinte verwendet man vorzugsweise eine wässrige Lösung eines wasserlöslichen Farbstoffes. Diese Farbstoffe haben generell eine geringere Lichtechtheit als die in konventionellen Druckverfahren verwendeten Farb-Pigmente. Als Folge davon sind im Tintenstrahldruck hergestellte Aufzeichnungen unter Lichtzutritt nur beschränkt lagerfähig.

Man hat daher bereits vorgeschlagen (US-A-4,256,493), der Tinte einen wasserlöslichen UV-Absorber vom Typ sulfonierter Hydroxybenzophenone zuzusetzen. Auch die Metallsalze solcher Verbindungen wurden als Lichtschutz-Additive für Tinten für den Tintenstrahldruck vorgeschlagen (JP-A-46277/88).

Solche Benzophenonderivate und deren Salze haben den Nachteil, dass sie mit bestimmten Farbstoffen, vor allem mit schwarzen Farbstoffen, Verfärbungen verursachen.

In Research Disclosure 22519 wurden auch schon Carbonsäurederivate von UV-Absorbern vom Benztriazol-Typ zur Stabilisierung von Farbstoffen und Tinten vorgeschlagen. Diese Derivate sind in wässrigen Systemen jedoch nicht genügend löslich. Aufzeichnungsmaterialien, die einen wasserlöslichen Stabilisator auf der Basis von Benztriazolen, Zintsäuren oder Triazinen enthalten, sind in der zwischen literatur EP-A-0 374 751 beschrieben. Die dort genannten Verbindungen unterscheiden sich von vorliegenden Anmeldungsgegenstand durch ein quartäres N-Atom.

Es wurde nunmehr gefunden, dass sich bestimmte wasserlösliche Derivate von 2-(2'-Hydroxyphenyl)-benztriazolen gut als Lichtschutz-Additive für wässrige Tinten eignen, insbesondere für den Tintenstrahldruck.

Die Erfindung betrifft wässrige Tinten, die als Lichtschutzmittel mindestens eine Verbindung der Formel I enthalten,

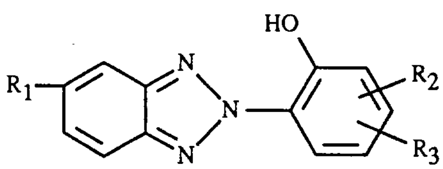

I

worin $R_1$ Wasserstoff, Halogen, $C_1$-$C_4$-Alkyl, $C_1$-$C_4$-Alkoxy, -COO-$R_9$-$OSO_3X$, -COOX oder -$SO_3X$ bedeutet,
$R_2$ Wasserstoff, OH, $C_1$-$C_8$-Alkyl, $C_5$-$C_6$-Cycloalkyl, Phenyl, Naphthyl, $C_7$-$C_{12}$-Aralkyl, Halogen, $C_1$-$C_8$-Alkoxy oder eine Gruppe -Y-Z bedeutet,
$R_3$ Wasserstoff, $C_1$-$C_8$-Alkyl, eine Gruppe -Y-Z bedeutet oder eine Gruppe -Y-O-Z',
X Wasserstoff oder M bedeutet,
M Li, Na, K, $\frac{1}{2}$ Mg, $\frac{1}{2}$ Ca, $\frac{1}{2}$ Zn, $\frac{1}{2}$ Co (II), $\frac{1}{2}$ Cu (II), $\frac{1}{2}$ Ni (II), $\frac{1}{3}$ Cr (III), $\frac{1}{3}$ Fe (III),

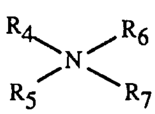 , 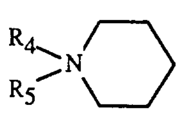 , 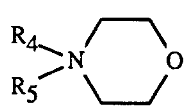 oder $\frac{1}{2}$ 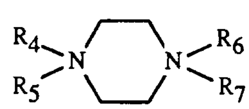

bedeutet, worin $R_4$, $R_5$, $R_6$ und $R_7$ unabhänig voneinander Wasserstoff, $C_1$-$C_{12}$-Alkyl, Hydroxyethyl, Cyclohexyl, Phenyl oder Benzyl bedeuten,
Y eine direkte Bindung ist oder eine zweiwertige Gruppe einer der folgenden Formeln bedeutet: -$R_8$-, -$R_8$-CONH-$R_9$-,

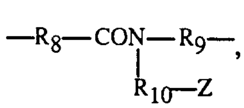 , 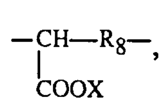 , 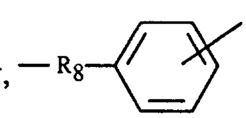 ,

$-R_8-COO-R_9-$, $-R_8-SO_2-R_9-$, $-R_8-SO_2NH-R_9-$,,

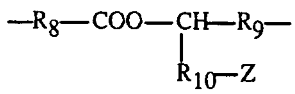 oder 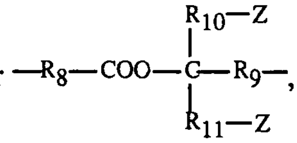,

worin $R_8$, $R_9$, $R_{10}$ und $R_{11}$ unabhängig voneinander $C_1$-$C_{20}$-Alkylen bedeuten, das durch 1-10 Hydroxylgruppen substituiert sein kann oder durch 1-10 -O- oder -NH- unterbrochenes $C_2$-$C_{20}$-Alkylen bedeuten und Z eine Gruppe -COOX oder $-SO_3X$ bedeutet, Z'-$SO_3X$ ist,
wobei die Verbindung der Formel I mindestens eine Gruppe der Formel -COOX oder $-SO_3X$ enthält.

$R_1$ als $C_1$-$C_4$-Alkyl kann z.B. Methyl, Ethyl, Propyl, Isopropyl, n-Butyl, sec.Butyl, Iso-butyl oder tert.Butyl sein. $R_2$ und $R_3$ als $C_1$-$C_8$-Alkyl können darüber hinaus auch z.B. n-Pentyl, sec.Pentyl, tert.Pentyl, n-Hexyl, Isohexyl, 2-Ethylbutyl, n-Heptyl, Isoheptyl, n-Octyl, 2-Ethylhexyl oder tert.Octyl sein. $R_4$-$R_7$ als $C_1$-$C_{12}$-Alkyl können darüber hinaus auch z.B. n-Decyl, n-Dodecyl oder i-Nonyl sein.

$R_2$ als Cycloalkyl oder Aryl kann z.B. Cyclopentyl, Cyclohexyl, Phenyl oder Naphthyl sein. $R_2$ als Aralkyl kann z.B. Benzyl, $\alpha$-Methylbenzyl, $\alpha,\alpha$-Dimethylbenzyl oder Naphthylmethyl sein.

$R_1$ als $C_1$-$C_4$-Alkoxy kann z.B. Methoxy, Ethoxy, Isopropoxy oder Butoxy sein. $R_2$ als $C_1$-$C_8$-Alkoxy kann darüber hinaus auch z.B. Pentyloxy, Hexyloxy, Heptyloxy oder Octyloxy sein.

$R_8$ als $C_1$-$C_8$-Alkylen kann unverzweigtes oder verzweigtes Alkylen sein, wie z.B. Methylen, Di-, Tri-, Tetra-, Penta-, Hexa- und Octamethylen, 1,2-Propylen oder 2-Methyl-tetramethylen. $R_9$, $R_{10}$ und $R_{11}$ als $C_1$-$C_{20}$-Alkylen können darüber hinaus auch z.B. Octa-, Deca-, Dodeca-, Hexadeca- oder Octadecamethylen sein. Bevorzugt sind $R_8$, $R_9$, $R_{10}$ und $R_{11}$ $C_1$-$C_4$-Alkylen.

$R_8$, $R_9$, $R_{10}$ und $R_{11}$ als durch -O- unterbrochenes Alkylen können z.B. 3-Oxatetramethylen, 3,6-Dioxaoctamethylen, 3,6,9,12-Tetraoxa-tetradecamethylen oder Reste der Formeln

$$-(OC_2H_4)_2- \text{ und } -(OC_2H_4)_6-$$

sein. $R_9$, $R_{10}$ und $R_{11}$ als durch OH substituiertes Alkylen kann insbesondere 2-Hydroxytrimethylen sein.

Bevorzugt enthält die Tinte als Lichtschutzmittel eine Verbindung der Formel I, worin $R_1$ Wasserstoff, Halogen, $C_1$-$C_4$-Alkyl, $C_1$-$C_4$-Alkoxy, $-COO-R_9-OSO_3X$, -COOX oder $-SO_3X$ bedeutet,
$R_2$ Wasserstoff, $C_1$-$C_8$-Alkyl, $C_7$-$C_9$-Phenylalkyl oder eine Gruppe -Y-Z bedeutet,
$R_3$ Wasserstoff, $C_1$-$C_4$-Alkyl, eine Gruppe -Y-Z oder eine Gruppe -Y-O-Z' bedeutet,
X Wasserstoff oder M bedeutet,
M Li, Na, K,

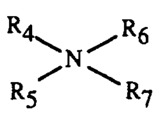, 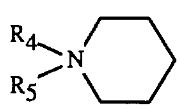 oder 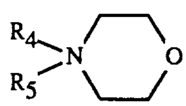

bedeutet, worin $R_4$, $R_5$,
$R_6$ und $R_7$ unabhängig voneinander H, $C_1$-$C_4$-Alkyl oder Hydroxyethyl bedeuten,
Y eine direkte Bindung oder eine der Gruppen $-R_8-$, $-R_8-CONH-R_9-$,

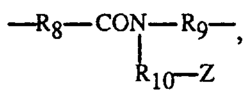,

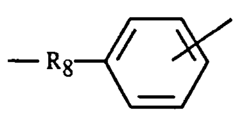,

$-R_8-COO-R_9-$ oder

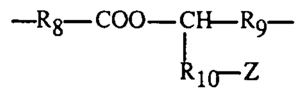

bedeutet,
worin $R_8$ $C_1$-$C_6$-Alkylen bedeutet und $R_9$ und $R_{10}$ unabhängig voneinander $C_1$-$C_{10}$-Alkylen bedeutet, das durch 1 oder 2 Hydroxylgruppen substituiert sein kann, oder durch 1-10 -O- unterbrochenes $C_2$-$C_{20}$-Alkylen bedeutet und
Z eine Gruppe -COOX oder $-SO_3X$ bedeutet und Z'-$SO_3X$ ist, insbesonders solche Verbindungen der Formel I,
worin $R_1$ Wasserstoff, Chlor,

$$-CO\!-\!(OC_2H_4)_x\!-\!OSO_3X,$$

-COOX oder $-SO_3X$ bedeutet,
$R_2$ Wasserstoff, $C_1$-$C_8$-Alkyl, $C_7$-$C_9$-Phenylalkyl oder eine Gruppe -Y-Z bedeutet,
$R_3$ Wasserstoff oder eine Gruppe -Y-Z bedeutet und x eine ganze Zahl von 2 bis 8 bedeutet.

Besonders bevorzugt sind Tinten, die eine Verbindung der Formel I enthalten, worin $R_1$ Wasserstoff, Chlor,

$$-CO\!-\!(OC_2H_4)_x\!-\!OSO_3X,$$

-COOX oder $-SO_3X$ bedeutet,
$R_2$ Wasserstoff, $C_1$-$C_8$-Alkyl, $C_7$-$C_9$-Phenylalkyl oder eine Gruppe -Y-Z bedeutet,
$R_3$ Wasserstoff oder eine Gruppe -Y-Z bedeutet, x eine ganze Zahl von 2 bis 8 ist,
X Li, Na, K oder

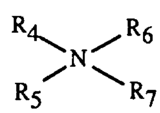

ist,
Y eine direkte Bindung oder eine der Gruppen $-R_8-$, $-R_8-CONH-R_9-$,

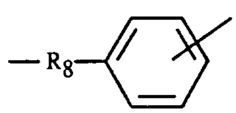

oder $-R_8-COO-R_9-$ bedeutet,
Z eine Gruppe -COOX oder $-SO_3X$ bedeutet,
$R_4$, $R_5$, $R_6$ und $R_7$ unabhängig voneinander Wasserstoff, $C_1$-$C_4$-Alkyl oder Hydroxyethyl bedeuten,
$R_8$ $C_1$-$C_6$-Alkylen bedeutet und $R_9$ $C_1$-$C_{10}$-Alkylen bedeutet, das durch 1 oder 2 Hydroxylgruppen substituiert sein kann, oder durch 1-10 -O- unterbrochenes $C_2$-$C_{20}$-Alkylen bedeutet.

X ist bevorzugt Li, Na, K, $NH_4$ oder Tetraalkylammonium, insbesondere Lithium.

Bevorzugt enthalten die Verbindungen der Formel I zwei Gruppen -COOX oder $-SO_3X$, insbesondere zwei Gruppen $-SO_3X$. Bevorzugt ist ferner Y eine direkte Bindung.

$R_2$ bzw. $R_3$ steht vorzugsweise in Ortho- bzw. Parastellung zur Hydroxylgruppe des Hydroxylphenylrestes in Formel I.

Beispiele für einzelne Verbindungen der Formel I sind:

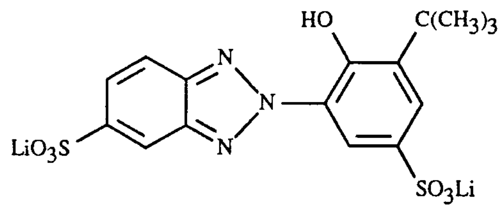

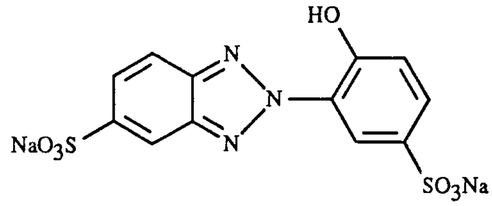

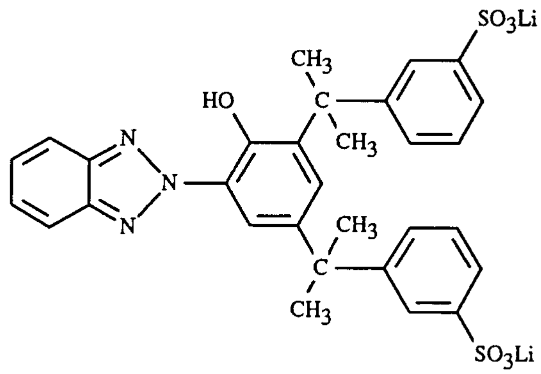

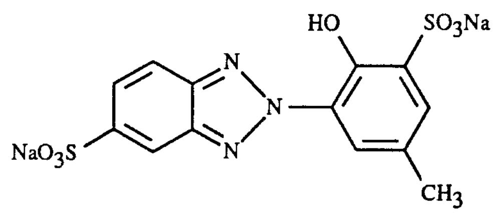

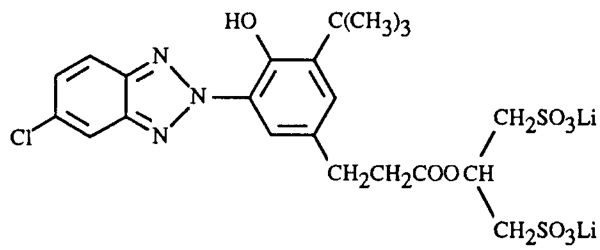

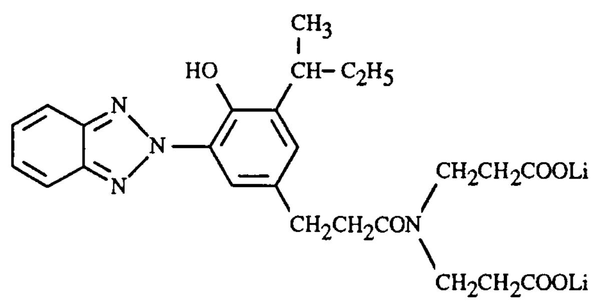

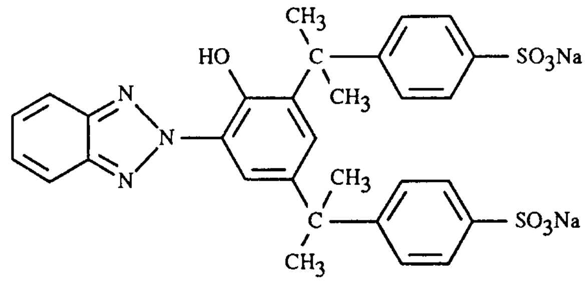

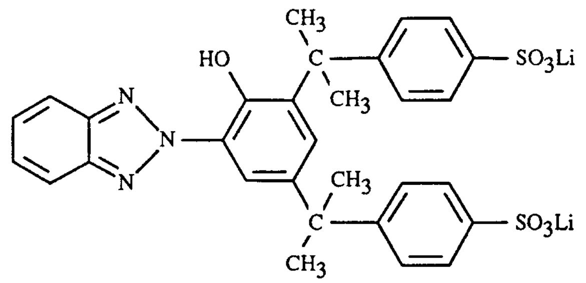

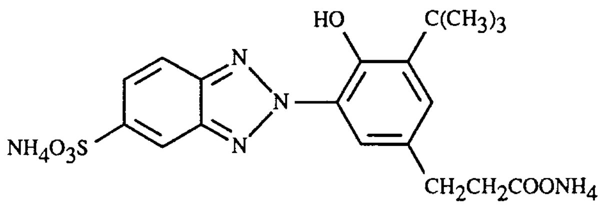

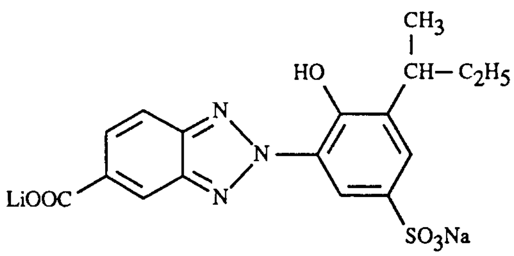

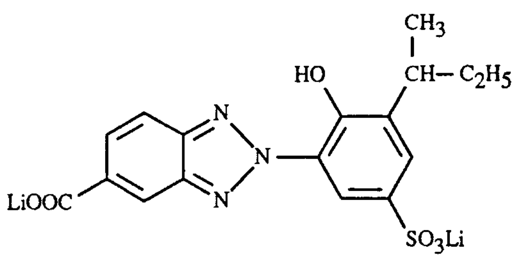

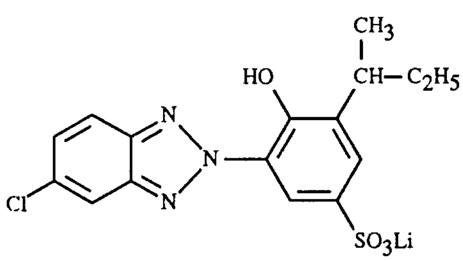

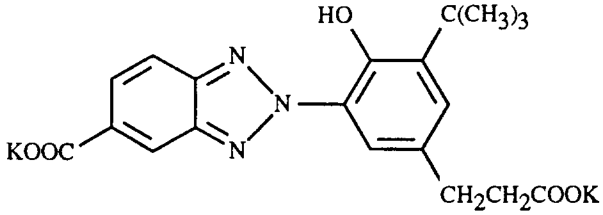

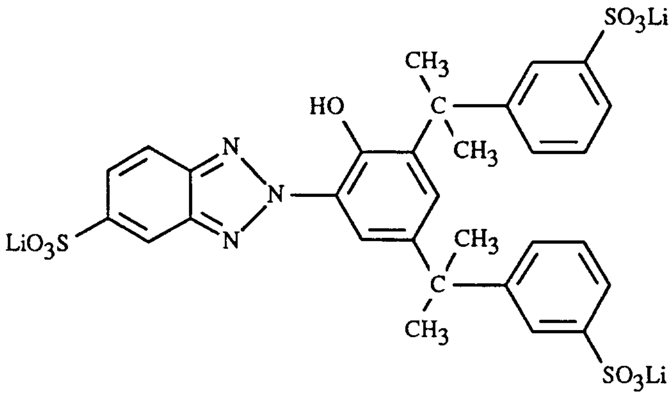

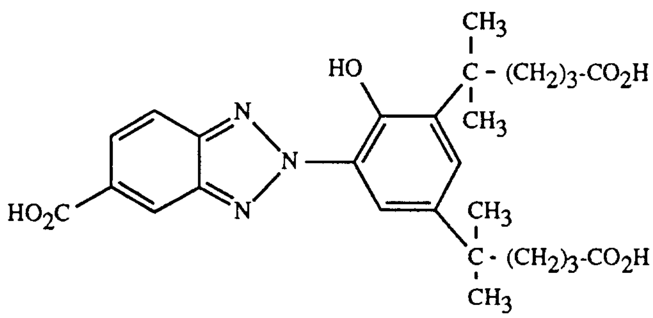 • $3N(CH_2CH_2OH)_3$

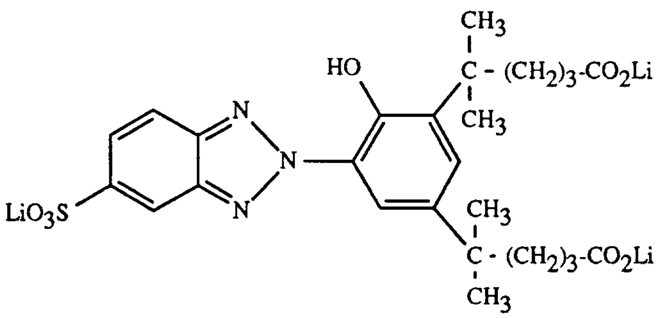

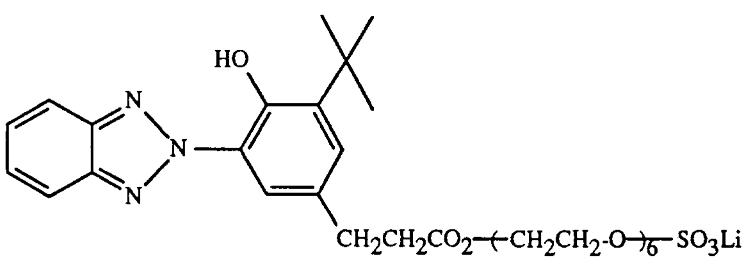

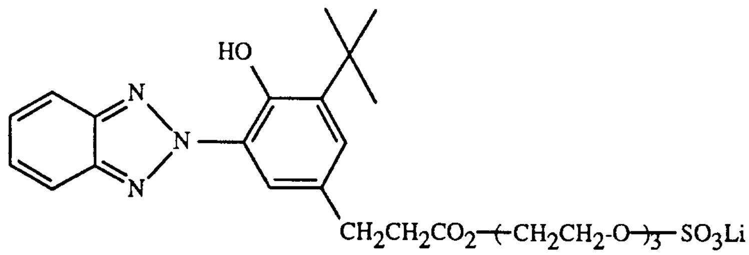

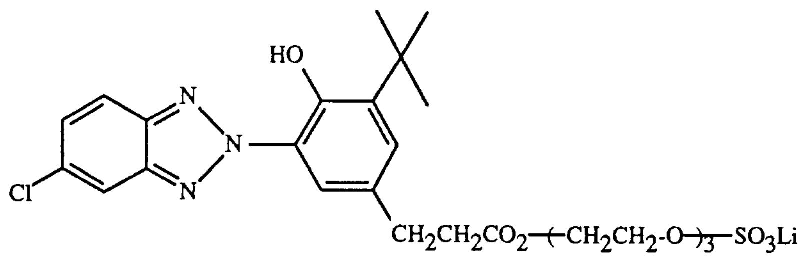

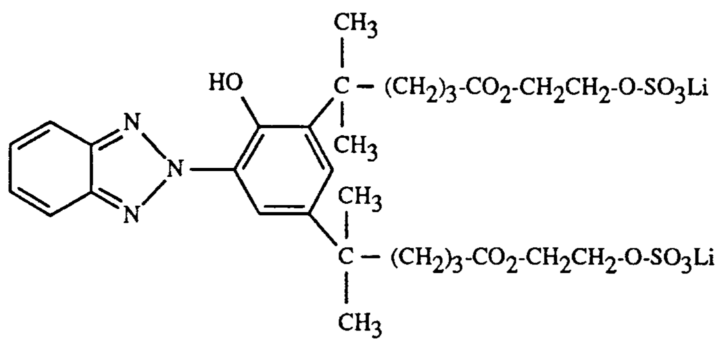

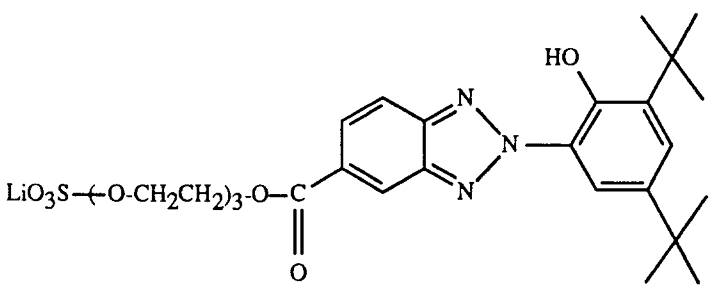

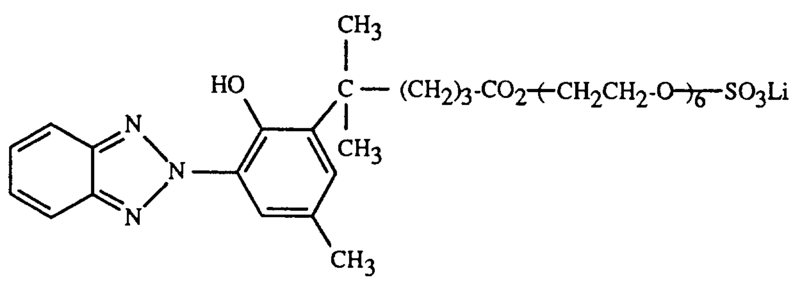

Die erfindungsgemäss verwendbaren Verbindungen enthalten mindestens eine Carbonsäure- oder Sulfonsäuregruppe bzw. deren Salze. Verbindungen mit zwei Carbonsäuregruppen sind bereits bekannt (GB-A-981,539, FR-A-1,370,874). Die meisten anderen Verbindungen sind neue Verbindungen, die ebenfalls Gegenstand der Erfindung sind.

Die Erfindung betrifft daher auch Verbindung der Formel I'

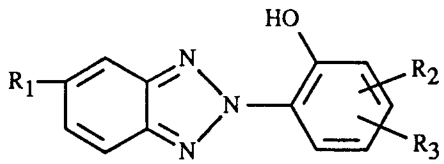

I'

worin $R_1$ Wasserstoff, Halogen, $C_1$-$C_4$-Alkyl, $C_1$-$C_4$-Alkoxy, -COO-$R_9$-$OSO_3X$, -COOM oder -$SO_3X$ bedeutet, $R_2$ Wasserstoff, OH, $C_1$-$C_8$-Alkyl, $C_5$-$C_6$-Cycloalkyl, Phenyl, Naphthyl, $C_7$-$C_{12}$-Aralkyl, Halogen, $C_1$-$C_8$-Alkoxy oder eine Gruppe -Y-Z bedeutet,

$R_3$ Wasserstoff, $C_1$-$C_8$-Alkyl oder eine Gruppe -Y-Z bedeutet, X Wasserstoff oder M bedeutet,

M Li, Na, K, $\frac{1}{2}$ Mg, $\frac{1}{2}$ Ca, $\frac{1}{2}$ Zn, $\frac{1}{2}$ Co (II), $\frac{1}{2}$ Cu (II), $\frac{1}{2}$ Ni (II), $\frac{1}{3}$ Cr (III), $\frac{1}{3}$ Fe (III),

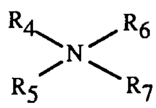, 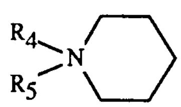, 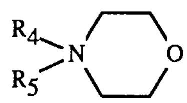 oder $\frac{1}{2}$ 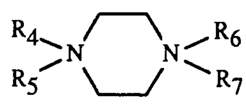

bedeutet, worin

$R_4$, $R_5$, $R_6$ und $R_7$ unabhänig voneinander Wasserstoff, $C_1$-$C_{12}$-Alkyl, Hydroxyethyl, Cyclohexyl, Phenyl oder Benzyl bedeuten,

X Wasserstoff oder M bedeutet,

Y eine der Gruppen -$R_8$-, -$R_8$-CONH-$R_9$-,

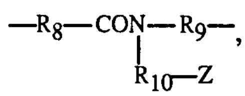, 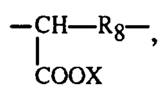,

-$R_8$-COO-$R_9$-, -$R_8$-$SO_2$-$R_9$-, -$R_8$-$SO_2$NH-$R_9$-,

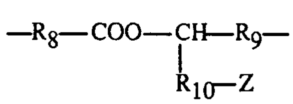 oder 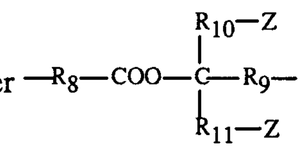

bedeutet,

worin $R_8$, $R_9$, $R_{10}$ und $R_{11}$ unabhängig voneinander $C_1$-$C_{20}$-Alkylen bedeuten, das durch 1-10 Hydroxylgruppen substituiert sein kann, oder durch 1-10 -O- oder -NH-unterbrochenes $C_2$-$C_{20}$-Alkylen bedeuten und Z eine Gruppe -COOM oder -$SO_3X$ bedeutet, wobei die Verbindung der Formel I' mindestens eine Gruppe -COOX und/oder mindestens eine Gruppe -$SO_3X$ enthält.

Diese Verbindungen können in Analogie zu anderen 2-(2-Hydroxyphenyl)-benztriazolen aus den Diazoniumsalzen der entsprechenden o-Nitroaniline durch Kupplung mit den entsprechenden substituierten Phenolen und cyclisierende Reduktion der gebildeten Azoverbindung hergestellt werden nach dem Schema

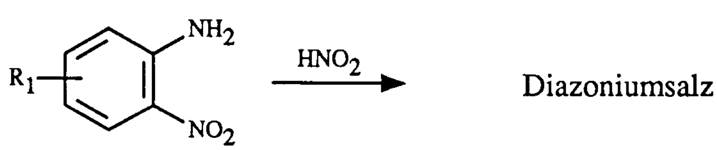

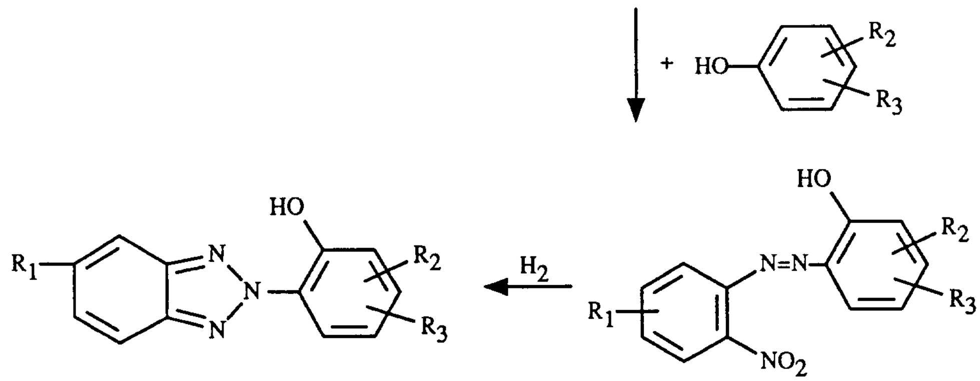


Alternativ kann ein Sulfonsäurerest als $R_1$, $R_2$ oder $R_3$ auch durch Sulfonierung des entsprechenden Benztriazoles eingeführt werden. Die Sulfonierung kann nach den üblichen Methoden der Aromaten-Sulfonierung durchgeführt werden, beispielsweise mit Chlorsulfonsäure. Eine weitere Alternative zur Einführung einer Sulfonsäuregruppe ist der Austausch einer tertiären Alkylgruppe als $R_2$ oder $R_3$ durch eine $SO_3H$-Gruppe durch Erwärmen in konzentrierter Schwefelsäure.

Bei diesen Synthesen fallen die Produkte meist als freie Carbon- bzw. Sulfonsäure an. Daraus können die entsprechenden Salze durch Neutralisation mit einer entsprechenden Base hergestellt werden, z.B. durch Neutralisation mit einem Hydroxid, Oxid, Carbonat, Ammoniak oder einem organischen Amin.

Bevorzugt sind solche Verbindungen der Formel I, die zwei Gruppen $-SO_3X$ enthalten.

Bevorzugt sind ferner Verbindungen der Formel I', worin $R_1$ Wasserstoff, Cl,

$$-CO-(OC_2H_4)_x-OSO_3X,$$

$-COOX$ oder $-SO_3X$ bedeutet,
$R_2$ Wasserstoff, $C_1$-$C_8$-Alkyl, $C_7$-$C_9$-Phenylalkyl oder eine Gruppe -Y-Z bedeutet,
$R_3$ Wasserstoff oder eine Gruppe -Y-Z bedeutet,
X Li, Na, K oder

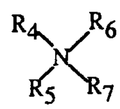

ist, x eine ganze Zahl von 2 bis 8 ist,
Y eine Gruppe $-R_8-$ oder $-R_8-COO-R_9-$ bedeutet, worin $R_8$ und $R_9$ $C_1$-$C_4$-Alkylen bedeuten,
und Z eine Gruppe $-COOX$ oder $-SO_3X$ ist, wobei in den Verbindungen der Formel I' mindestens zwei Gruppen $-SO_3X$ enthalten sind.

In bevorzugten Verbindungen der Formel I' befindet sich der Substituent $R_2$ bzw. $R_3$ in Ortho- bzw. Parastellung zur Hydroxylgruppe des Hydroxylphenylrestes.

Die Verbindungen der Formel I werden den Tinten vorzugsweise in einer Menge von 0,01 bis 20 Gew.-%, vorzugsweise von 0,1-10 Gew.-%, zugesetzt. Sie sind in diesen Konzentrationen gut löslich.

Die Tinten sind wässrige Tinten. Sie enthalten mindestens 30 Gew.-% Wasser. Neben Wasser können sie (bis zu 70 Gew.-%) noch ein oder mehrere mit Wasser mischbare Lösungsmittel enthalten, wie z.B. Ethylenglykol, Di- oder Triethylenglykol, Polyethylenglykol, Propylenglykol oder Ether solcher Glykole, Butandiol-1,4, Thiodiglykol, Glycerin und dessen Ether, Polyglycerin, Mono-, Di- und Triethanolamin, Propanolamin, Dimethylformamid, Dimethylsulfoxid, Dimethylacetamid, N-Methylpyrrolidon, Methanol, Ethanol, Isopropanol, n-Propanol, Diacetonalkohol, Aceton, Methylethylketon oder Propylencarbonat.

Bevorzugt enthalten die Tinten mindestens 50 Gew.-% Wasser.

Die Tinten enthalten wasserlösliche Farbstoffe oder Mischungen von Farbstoffen, wie sie für das Färben von natürlichen Fasern bekannt sind. Dies können z.B. Mono-, Di- oder Polyazofarbstoffe sein, Triphenylmethanfarbstoffe, Reaktivfarbstoffe oder Phthalocyaninfarbstoffe. Beispiele für solche Farbstoffe sind die im Colour Index genannten Farbstoffe Acid Red 14, Acid Red 52, Reactive Red 40, Acid Yellow 17, Acid

Yellow 23, Direct Yellow 86, Acid Red 35, Acid Red 249, Direct Red 227, Acid Blue 9, Direct Blue 86, Direct Blue 199, Food Black 2, Direct Black 19, Direct Black 38, Direct Black 168 und Sulphur Black 1. Im allgemeinen enthalten die Tinten 0,5-6 Gew.-% Farbstoff.

Die Tinten können auch verschiedene Zusätze in kleineren Mengen enthalten, wie z.B. Bindemittel, Tenside, Waschechtheitsverbesserer, Biocide, Korrosionsinhibitoren, Sequestriermittel, pH-Puffer oder Leitfähigkeitszusätze. Sie können auch weitere wasserlösliche Lichtschutzmittel enthalten. Im allgemeinen genügt jedoch die erfindungsgemässe Zugabe eines oder mehrerer Stabilisatoren der Formel I zur Tinte.

Die erfindungsgemäss stabilisierten Tinten werden bevorzugt für den Tintenstrahldruck verwendet. Sie können jedoch auch für alle sonstigen für Tinten üblichen Verwendungen eingesetzt werden, wie z.B. für Filzstifte, Stempelkissen, Füllfedern, Federschreiber (Pen Plotters), Farbbänder oder für verschiedene Druckverfahren als Druckfarben.

Beim Tintenstrahl-Druck gibt es verschiedene Verfahren in Abhängigkeit von der verwendeten Apparatur. So gibt es z.B. drop-on-demand printers, bubble-jet printers oder continuous-jet printers. Für alle diese apparativen Verfahren lassen sich die erfindungsgemäss stabilisierten Tinten verwenden.

Der Effekt des Zusatzes einer Verbindung der Formel I zu den Tinten besteht in der höheren Lichtbeständigkeit des mit der Tinte erzeugten Druckes. Die experimentelle Messung des Effektes kann durch Schnellbelichtung von Probedrucken in einem Belichtungsgerät geschehen, wie es in den folgenden Beispielen gezeigt wird. Teile bedeuten darin Gewichtsteile und % bedeuten Gewichts-%.

Die erfindungsgemässen Aufzeichnungsmaterialien, die vorzugsweise für das Tintenstrahldruckverfahren verwendet werden, bestehen aus einem Träger mit einer durch Tintenstrahl bedruckbaren Oberfläche. Der Träger ist üblicherweise Papier oder eine Plastikfolie und normalerweise auf einer Seite mit einem Material beschichtet, das für Tinten aufnahmefähig ist. Vorzugsweise enthält diese Schicht $SiO_2$ und Polyvinylalkohol.

Unbeschichtetes Papier kann ebenfalls eingesetzt werden. Hier dient das Papier gleichzeitig als Trägermaterial und Tintenaufnahmeschicht. Ferner lassen sich auch Materialien aus Cellulosefasern und textile Fasermaterialien, beispielsweise Baumwollgewebe oder Baumwollmischgewebe aus Baumwolle und Polyacrylamid oder Polyester, welche Verbindungen der Formel (I) enthalten, für den Tintenstrahldruck verwenden.

Die Aufzeichnungsmaterialien können auch transparent sein, wie im Falle von Projektionsfolien.

Die Verbindungen der Formel I können auch in Aufzeichnungsmaterialien, insbesondere für den Tintenstrahldruck eingearbeitet werden.

In einer ersten Applikationsmethode können die Verbindungen der Formel I direkt dem Papierbrei bei der Papierherstellung zugesetzt werden.

Eine zweite Applikationsmethode ist das Besprühen des Trägermaterials mit einer Lösung der Verbindungen der Formel (I). Hierbei handelt es sich um eine wässrige Lösung oder eine Lösung in einem leicht flüchtigen organischen Lösungsmittel. Speziell im Falle öllöslicher Verbindungen der Formel (I) eignet sich das Aufsprühen von bzw. Tränken des Materials mit einer organischen Lösung einer Verbindung der Formel (I). Des weiteren bietet sich die Verwendung von Emulsionen bzw. Dispersionen an.

Meist wird jedoch eine farbstoffaffine Beschichtungsmasse auf das Trägermaterial aufgebracht, und in diesem Fall setzt man die Verbindungen der Formel (I) dieser Beschichtungsmasse zu. Die Beschichtungsmassen bestehen in der Regel aus einem festen Füllstoff, einem Bindemittel sowie üblichen Additiven.

Der Füllstoff ist mengenmässig der Hauptbestandteil der Beschichtungsmasse. Beispiele für Füllstoffe, die in Frage kommen, sind $SiO_2$, Kaolin, Talk, Ton, Ca-, Mg- oder Al-Silikate, Gips, Zeolith, Bentonit, Diatomenerde, Vemiculit, Stärke oder das in JP-A-60-260 377 beschriebene oberflächenmodifizierte $SiO_2$. Geringe Mengen an weissen Pigmenten, wie z.B. Titandioxid, Baryt, Magnesiumoxid, Kalk, Kreide oder Magnesiumcarbonat können mit dem Füllstoff in der Beschichtungsmasse verwendet werden, sofern sie die Dichte des Tintenstrahldrucks nicht stark herabsetzen.

Beschichtungsmassen, die für transparente, projektionsfähige Aufzeichnungsmaterialien bestimmt sind, können keine Licht streuende Teilchen, wie Pigmente und Füllstoffe, enthalten.

Das Bindemittel bindet die Füllstoffe unter sich und an das Trägermaterial. Beispiele für gebräuchliche Bindemittel sind wasserlösliche Polymere, wie z.B. Polyvinylalkohol, partiell hydrolysiertes Polyvinylacetat, Cellulose-ether, Polyvinylpyrrolidon und dessen Copolymere, Polyethylenoxid, Salze von Polyacrylsäure, Natrium-alginat, oxidierte Stärke, Gelatine, Casein, Pflanzengummi, Dextrin, Albumin, Dispersionen und Polyacrylaten oder Acrylat-Methacrylat-Copolymeren, Latices von Natur- oder Synthesekautschuk, Poly-(meth)acrylamid, Polyvinylether, Polyvinylester, Copolymere von Maleinsäure, Melaminharze, Harnstoffharze oder chemisch modifizierte Polyvinylalkohole, wie in JP-A-61-134 290 oder JP-A-61-134 291 beschrieben.

Man kann dem Bindemittel einen zusätzlichen Farbstoffrezeptor oder ein Beizmittel zusetzen, die die Fixierung des Farbstoffs an die Beschichtung verbessern. Farbstoffrezeptoren für saure Farbstoffe sind

kationischer oder amphoterer Natur. Beispiele für kationische Rezeptoren sind polymere Ammoniumverbindungen wie z.B. Polyvinylbenzyl-trimethylammoniumchlorid, Polydiallyl-dimethylammoniumchlorid, Polymethacryloxyethyl-dimethylhydroxyethylammonium-chlorid, Polyvinylbenzyl-methylimidazolium-chlorid, Polyvinylbenzyl-picoliniumchlorid oder Polyvinylbenzyl-tributylammoniumchlorid. Weitere Beispiele sind basische Polymere wie z.B. Poly-(dimethylaminoethyl)methacrylat, Polyalkylenpolyamine und deren Kondensationsprodukte mit Dicyandiamid, Amin-Epichlorhydrin-Polykondensate oder die in den JP-A-57-36 692, 57-64 591, 57-187 289, 57-191 084, 58-177 390, 58-208 357, 59-20 696, 59-33 176, 59-96 987, 59-198 188, 60-49 990, 60-71 796, 60-72 785, 60-161 188, 60-187 582, 60-189 481, 60-189 482, 61-14 979, 61-43 593, 61-57 379, 61-57 380, 61-58 788, 61-61 887, 61-63 477, 61-72 581, 61-95 977, 61-134 291 oder in den US-A-4 547 405 und 4 554 181 sowie in der DE-A-3 417 582 beschriebenen Verbindungen. Ein Beispiel für amphotere Farbstoff-Rezeptoren ist Gelatine.

Die farbstoffaffine Beschichtung kann eine Reihe weiterer Additive enthalten, wie z.B. Antioxidantien, weitere Lichtschutzmittel (darunter auch UV-Absorber, die nicht den erfindungsgemässen Lichtschutzmittel entsprechen), Viskositätsverbesserer, optische Aufheller, Biocide und/oder Antistatika.

Beispiele für geeignete Antioxidantien sind insbesondere sterisch gehinderte Phenole und Hydrochinone, wie z.B. die in GB-A-2 088 777, oder den JP-A-60-72 785, 60-72 786 und 60-71 796 aufgeführten Antioxidantien.

Beispiele für geeignete Lichtschutzmittel sind insbesondere organische Nickelverbindungen und sterisch gehinderte Amine, wie z.B. die in den JP-A-58-152 072, 61-146 591, 61-163 886, 60-72 785 und 61-146 591 oder die in der GB-A-2 088 777, JP 59-169 883 und 61-177 279 erwähnten Lichtschutzmittel.

Geeignete UV-Absorber, die in Kombination mit Verbindungen der Formel (I) einer Beschichtungsmasse zugesetzt werden können, sind z.B. in Research Disclosure Nr. 24239 (1984) Seite 284, GB-A-2 088 777 und EP-A-0 280 650 beschrieben. Besonders die UV-Absorber der 2-Hydroxyphenylbenztriazol-Klasse und ganz besonders 2-(2'-Hydroxy-3',5'-di-t-amylphenyl)-benzotriazol und 2-(2'-Hydroxy-3'-t-butyl-5'-polyglykolpropionat-phenyl)-benztriazol sind für die Verwendung gemeinsam mit Verbindungen der Formel (I) in Aufzeichungsmaterialien für den Tintenstrahldruck geeignet. Die UV-Absorber können als Emulsion oder als Dispersion der Beschichtungsmasse zugegeben werden. Handelt es sich bei der Verbindung der Formel (I) um eine Säure, so kann diese durch Zugabe einer Lauge in der Beschichtungsmasse gelöst werden. Verbindungen der Formel (I), die keine Säuren sind, können entweder direkt in der Beschichtungsmasse gelöst werden oder ihr in Form einer Emulsion oder Suspension zugesetzt werden.

Die Beschichtungsmasse wird in der Regel auf den Träger, z.B. Papier, aufgetragen und durch Erhitzen getrocknet. Die Verbindungen der Formel (I) können, wie bereits erwähnt, auch in einem separaten Arbeitsgang, allein oder zusammen mit anderen, bereits beschriebenen Komponenten, als wässrige Lösung auf das Aufzeichnungsmaterial gebracht werden. Das Aufbringen kann durch Besprühen, Verleimen in einer Verleimungspresse, einem getrennten Giessvorgang oder durch Eintauchen in eine Wanne erfolgen. Nach einer solchen Nachbehandlung des Aufzeichnungsmaterials ist ein zusätzlicher Trocknungsvorgang nötig.

Das Aufzeichnungsmaterial enthält vorzugsweise 1 bis 10000 mg/m$^2$, insbesondere 50 bis 2000 mg$^2$, mindestens einer Verbindung der Formel (I).

Beispiel 1: Eine Beschichtungsmasse auf der Basis von Silika/Polyvinylalkohol wird aus den folgenden Komponenten hergestellt:

16,34 g einer 10%-igen Lösung von Polyvinylalkohol (Riedel de Haen GmbH)
0,02 g Di-tert-octylphenyl-polyethylenoxid
2,00 g Silika (Typ 244 W.R. Grace and Co.)
9,54 g Wasser

Die resultierende Beschichtungsmasse wird mit Hilfe von Ultraschall dispergiert und durch ein Sieb aus Polyesterfasern mit der Maschenweite 24 µm filtriert. Der pH wird durch Zusatz von 2N Natronlauge auf 7,0 eingestellt.

Die Beschichtungsmasse wird mit einer Drahtspirale auf photographisches Papier in einer Dicke von 36 µm aufgetragen. Nach dem Trocknen mit warmer Luft beträgt der Trockenauftrag der Beschichtung etwa 5,0 g/m$^2$.

Das Aufzeichnungsmaterial wird jeweils mit einer erfindungsgemässen Tinte, welche einen UV-Absorber der Formel I enthält und einer Vergleichstinte, die keinen UV-Absorber enthält, bedruckt.

Die Tinte dazu wird wie folgt hergestellt:

Man löst 3 g eines UV-Absorbers der Formel

(UV-I)

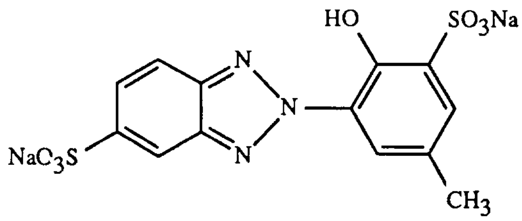

in einer Mischung aus 83 g Wasser und 15 g Glycerin. Eine Farbstoff-Lösung wird bereitet aus 4 g C.I. Food Black 2 in 80 g Wasser und 15 g Glycerin. Beide Lösungen werden durch einen Ultrafilter mit 0,3 µm Porenweite filtriert und vereinigt. Die erhaltene Drucktinte besteht aus:

81,5 % Wasser
15 % Glycerin
2 % Farbstoff
1,5 % UV-Absorber

Die Blindprobe wird durch Vereinigung der Farbstofflösung mit gleichen Teilen eines Gemisches von 86 g Wasser und 15 g Glycerin bereitet.

Die Tinten werden in Tintenpatronen des Tintenstrahldruckers "Think-Jet" der Fa. Hewlett Packard gefüllt. Es werden bedruckte Proben mit einer Punktdichte von 192x96 Punkte pro Inch (75,6x37,8 Punkte pro $cm^2$) hergestellt.

Nach einer Woche Lagerung, um den Druck völlig auszutrocknen, wird die Farbdichte (Intensität) der bedruckten Proben mit einem Densitometer (Macbeth TR 924) unter Verwendung eines Status A-Filters gemessen. Dann werden die Probedrucke in einem Atlas Weather-o-meter mit einer Xenonlampe einer Beleuchtungsstärke von 81 kLux hinter einem Filter aus 6 mm dickem Fensterglas bestrahlt. Anschliessend wird erneut die Farbdichte gemessen, um den prozentualen Verlust an Farbdichte zu ermitteln.

Die Ergebnisse sind in der nachfolgenden Tabelle zusammengefasst. Niedrigere Werte bedeuten höhere Lichtechtheit.

| UV-Absorber | Farbdichteverlust nach Belichtung |
|---|---|
| keiner | 15 % |
| 1,5 % | 10 % |

Macht man denselben Versuch unter Verwendung von 2-Hydroxy-4-methoxybenzophenon-5-sulfonsaurem Natrium als UV-Absorber, so verfärbt sich der schwarze Druck nach braunschwarz.

Beispiel 2: Ein Tintenkonzentrat aus 4 g C.I. Acid Red 249, 25 g Diäthylenglykol und 70 g Wasser sowie ein UV-Absorberkonzentrat aus 4 g UV-Absorber, 25 g Diäthylenglykol und 70 g Wasser wurden hergestellt. Als UV-Absorber wurden die Verbindungen

UV-II

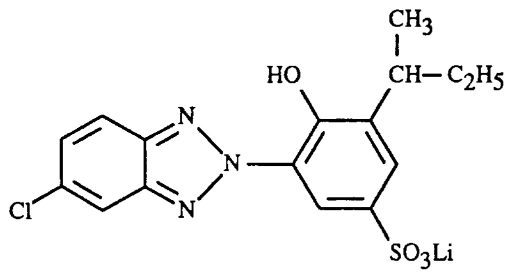

und

UV-III

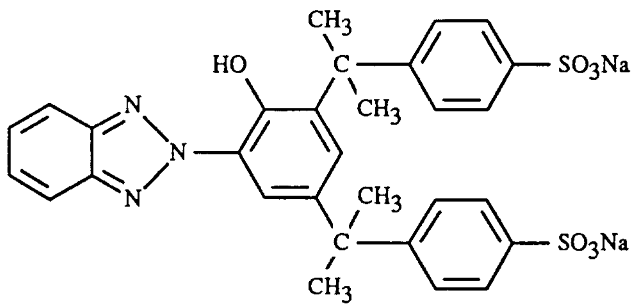

sowie als Vergleichs-UV-absorber die Verbindungen der Formeln

V-I

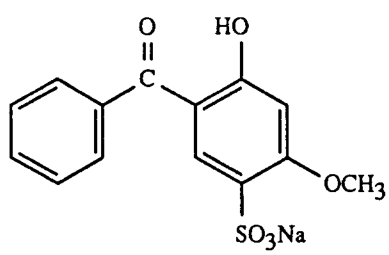

und

V-II

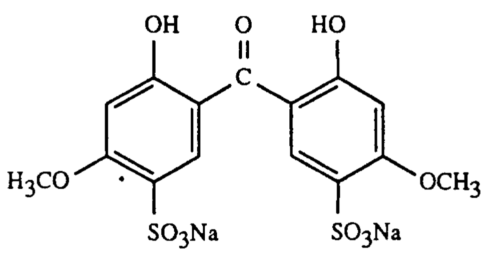

eingesetzt.

Aus je 1 Teil Farbstoffkonzentrat und UV-Absorberkonzentrat wurden Tinten hergestellt und in Patronen eines Hewlett Packard Quiet-Jet-Drucker gefüllt. Nach Anfertigung von Probedrucken auf für den Tintenstrahldruck geeignetes Papier wurde wie in Beispiel 1 beschrieben die Lichtechtheit dieser Drucke geprüft. Die Resultate sind in Tabelle 1 zusammengefasst.

Tabelle 1

| UV-Absorber | Dichteverlust [%] ($15\ kJ/cm^2$) |
|---|---|
| ohne | 63 |
| UV-II | 50 |
| UV-III | 46 |
| V-I | 59 |
| V-II | 69 |

Beispiel 3: Wie in Beispiel 2 beschrieben wurden mit den Farbstoffen Food Black 2, Acid Red 14, 27, 35 und 249, Direct Red 227 und Reactive Red 24 Tinten hergestellt, wobei das UV-Absorberkonzentrat jedoch aus 6 g UV-Absorber, 25 g Diäthylenglykol und 70 g Wasser bestand.

Das Konzentrat mit Reactive Red 24 wurde vor Verwendung mit Lithiumhydroxid auf einen pH-Wert von 12,0 eingestellt, 30 Minuten lang bei 95 bis 100 ° C erhitzt und danach mit Schwefelsäure auf einen pH-Wert von 7 gestellt.

Die Anfertigung von Drucken erfolgte auf die in Beispiel 2 angegebene Weise, ebenso die Prüfung auf Lichtechtheit. Die Resultate sind in Tabelle 2 zusammengefasst.

Tabelle 2:

| UV-Absorber | Dichteverlust in % nach 15 kJ/cm² Bestrahlung | | | | | | |
|---|---|---|---|---|---|---|---|
| | Food Black 2 | Acid Red 249 | Direct Red 227 | Acid Red 14 | Acid Red 35 | Reactive Red 24 | Acid Red 27 |
| ohne | 53 | 63 | 72 | 90 | 75 | 54 | 81 |
| UV-I | 35 | 45 | 57 | 78 | 65 | 37 | 63 |
| V-I | 47 | 55 | 78 | 85 | 80 | 57 | 78 |
| V-II | 57 | 78 | 72 | 91 | 92 | 66 | 88 |

UV-I entspricht der Verbindung der Formel

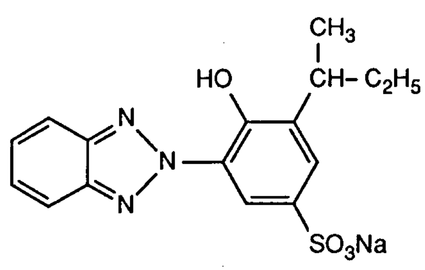

## Patentansprüche

1. Wässrige Tinte, enthaltend als Lichtschutzmittel mindestens eine Verbindung der Formel I,

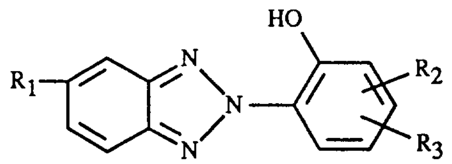

I

worin $R_1$ Wasserstoff, Halogen, $C_1$-$C_4$-Alkyl, $C_1$-$C_4$-Alkoxy, -COO-$R_9$-$OSO_3X$, -COOX oder -$SO_3X$ bedeutet,
$R_2$ Wasserstoff, OH, $C_1$-$C_8$-Alkyl, $C_5$-$C_6$-Cycloalkyl, Phenyl, Naphthyl, $C_7$-$C_{12}$-Aralkyl, Halogen, $C_1$-$C_8$-Alkoxy oder eine Gruppe -Y-Z bedeutet,
$R_3$ Wasserstoff, $C_1$-$C_8$-Alkyl, eine Gruppe -Y-Z oder eine Gruppe -Y-O-Z' bedeutet, X Wasserstoff oder M bedeutet,
M Li, Na, K, $\frac{1}{2}$ Mg, $\frac{1}{2}$ Ca, $\frac{1}{2}$ Zn, $\frac{1}{2}$ Co (II), $\frac{1}{2}$ Cu (II), $\frac{1}{2}$ Ni (II), $\frac{1}{3}$ Cr (III), $\frac{1}{3}$ Fe (III),

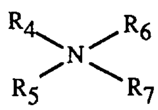 , 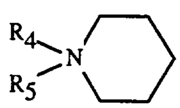 , 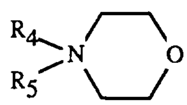 oder $\frac{1}{2}$ 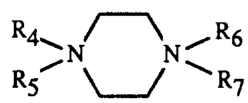

bedeutet, worin
$R_4$, $R_5$, $R_6$ und $R_7$ unabhänig voneinander Wasserstoff, $C_1$-$C_{12}$-Alkyl, Hydroxyethyl, Cyclohexyl, Phenyl oder Benzyl bedeuten,
Y eine direkte Bindung ist oder eine zweiwertige Gruppe einer der folgenden Formeln bedeutet:
-$R_8$-, -$R_8$-CONH-$R_9$-,

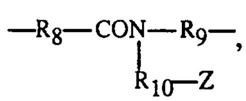 , 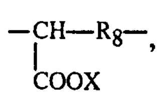 , 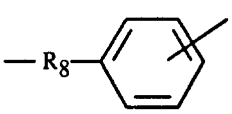 ,

-$R_8$-COO-$R_9$-, -$R_8$-$SO_2$-$R_9$-, -$R_8$-$SO_2$NH-$R_9$-,,

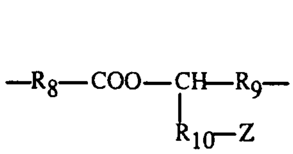 oder 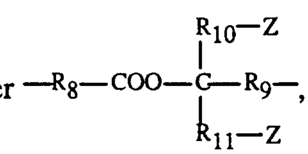 ,

worin $R_8$, $R_9$, $R_{10}$ und $R_{11}$ unabhängig voneinander $C_1$-$C_{20}$-Alkylen bedeuten, das durch 1-10 Hydroxylgruppen substituiert sein kann oder durch 1-10 -O- oder -NH- unterbrochenes $C_2$-$C_{20}$-Alkylen bedeuten und
Z eine Gruppe -COOX oder -$SO_3X$ bedeutet, Z'-$SO_3X$ ist,
wobei die Verbindung der Formel I mindestens eine Gruppe der Formel -COOX oder -$SO_3X$ enthält.

2. Tinte gemäss Anspruch 1, enthaltend eine Verbindung der Formel I, worin $R_1$ Wasserstoff, Halogen, $C_1$-$C_4$-Alkyl, $C_1$-$C_4$-Alkoxy, -COO-$R_9$-$OSO_3X$, -COOX oder -$SO_3X$ bedeutet,
$R_2$ Wasserstoff, $C_1$-$C_8$-Alkyl, $C_7$-$C_9$-Phenylalkyl, eine Gruppe -Y-Z bedeutet,
$R_3$ Wasserstoff, $C_1$-$C_4$-Alkyl, eine Gruppe -Y-Z oder eine Gruppe -Y-O-Z' bedeutet,
X Wasserstoff oder M bedeutet, M Li, Na, K,

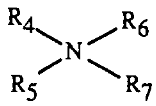, 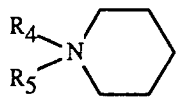 oder 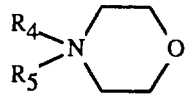

bedeutet, worin $R_4$, $R_5$, $R_6$ und $R_7$ unabhängig voneinander H, $C_1$-$C_4$-Alkyl oder Hydroxyethyl bedeuten, Y eine direkte Bindung oder eine der Gruppen $-R_8-$, $-R_8-CONH-R_9-$,

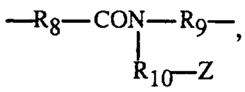

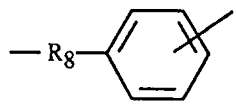,

$-R_8-COO-R_9-$ oder

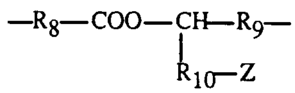

bedeutet,
worin $R_8$ $C_1$-$C_6$-Alkylen bedeutet und $R_9$ und $R_{10}$ unabhängig voneinander $C_1$-$C_{10}$-Alkylen bedeutet, das durch 1 oder 2 Hydroxylgruppen substituiert sein kann oder durch 1-10 -O- unterbrochenes $C_2$-$C_{20}$-Alkylen bedeutet,
Z eine Gruppe -COOX oder $-SO_3X$ bedeutet und $Z'-SO_3X$ ist.

**3.** Tinte gemäss Anspruch 2, enthaltend eine Verbindung der Formel I, worin $R_1$ Wasserstoff, Chlor,

$$-CO-(OC_2H_4)_x-OSO_3X,$$

-COOX oder $-SO_3X$ bedeutet,
$R_2$ Wasserstoff, $C_1$-$C_8$-Alkyl, $C_7$-$C_9$-Phenylalkyl oder eine Gruppe -Y-Z bedeutet,
$R_3$ Wasserstoff oder eine Gruppe -Y-Z bedeutet, x eine ganze Zahl von 2 bis 8 ist und M, X, Y und Z die in Anspruch 2 gegebene Bedeutung haben.

**4.** Tinte gemäss Anspruch 3, enthaltend eine Verbindung der Formel I, worin $R_1$, $R_2$ und $R_3$ die in Anspruch 3 gegebene Bedeutung haben, X Li, Na, K oder

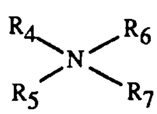

ist,
Y eine direkte Bindung oder eine der Gruppen $-R_8-$, $-R_8-CONH-R_9-$,

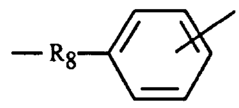

oder $-R_8-COO-R_9-$ bedeutet,
Z eine Gruppe $-COOX$ oder $-SO_3X$ bedeutet, und $R_4$, $R_5$, $R_6$, $R_7$, $R_8$ und $R_9$ die in Anspruch 2 gegebene Bedeutung haben.

**5.** Tinte gemäss Anspruch 1, enthaltend eine Verbindung der Formel I, worin $R_2$ in Orthostellung und $R_3$ in Parastellung zur OH-Gruppe stehen.

**6.** Tinte gemäss Anspruch 1, enthaltend 0,01-20 Gew.-% mindestens einer Verbindung der Formel I.

**7.** Tinte gemäss Anspruch 1, enthaltend 0,1-10 Gew.-% mindestens einer Verbindung der Formel I.

**8.** Tinte gemäss Anspruch 1, enthaltend mindestens 30 Gew.-% Wasser.

**9.** Verwendung einer Tinte gemäss Anspruch 1 für den Tintenstrahldruck.

**10.** Verbindungen der Formel I,'

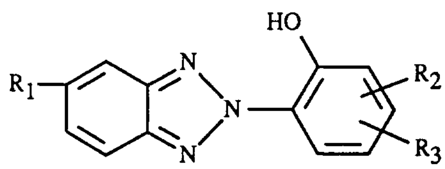

I'

worin $R_1$ Wasserstoff, Halogen, $C_1$-$C_4$-Alkyl, $C_1$-$C_4$-Alkoxy, $-COO-R_9-OSO_3X$, $-COOM$ oder $-SO_3X$ bedeutet,
$R_2$ Wasserstoff, OH, $C_1$-$C_8$-Alkyl, $C_5$-$C_6$-Cycloalkyl, Phenyl, Naphthyl, $C_7$-$C_{12}$-Aralkyl, Halogen, $C_1$-$C_8$-Alkoxy oder eine Gruppe -Y-Z bedeutet,
$R_3$ Wasserstoff, $C_1$-$C_8$-Alkyl oder eine Gruppe -Y-Z bedeutet,
X Wasserstoff oder M bedeutet,
M Li, Na, K, $\frac{1}{2}$ Mg, $\frac{1}{2}$ Ca, $\frac{1}{2}$ Zn, $\frac{1}{2}$ Co (II), $\frac{1}{2}$ Cu (II), $\frac{1}{2}$ Ni (II), $\frac{1}{3}$ Cr (III), $\frac{1}{3}$ Fe (III),

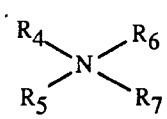 , 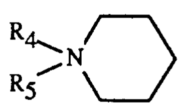 , 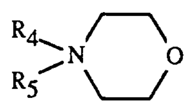 oder $\frac{1}{2}$ 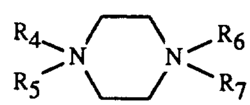

bedeutet, worin $R_4$, $R_5$, $R_6$ und $R_7$ unabhänig voneinander Wasserstoff, $C_1$-$C_{12}$-Alkyl, Hydroxyethyl, Cyclohexyl, Phenyl oder Benzyl bedeuten,
Y eine der Gruppen $-R_8-$, $-R_8-CONH-R_9-$,

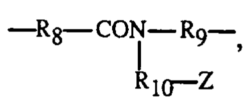 , 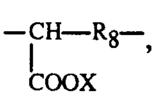 ,

$-R_8-COO-R_9-$, $-R_8-SO_2-R_9-$, $-R_8-SO_2NH-R_9-$,

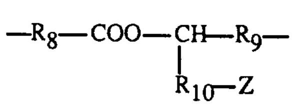 oder 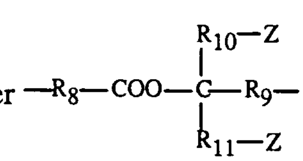

bedeutet, worin $R_8$, $R_9$, $R_{10}$ und $R_{11}$ unabhängig voneinander $C_1$-$C_{20}$-Alkylen bedeuten, das durch 1-10 Hydroxylgruppen substituiert sein kann, oder durch 1-10 -O- oder -NH-unterbrochenes $C_2$-$C_{20}$-Alkylen bedeuten
und Z eine Gruppe -COOM oder -$SO_3X$ bedeutet, wobei die Verbindung der Formel I' mindestens eine Gruppe -COOX und/oder mindestens eine Gruppe -$SO_3X$ enthält.

**11.** Verbindungen gemäss Anspruch 9, die zwei Gruppen -$SO_3X$ enthalten.

**12.** Verbindungen gemäss Anspruch 11, worin $R_1$ Wasserstoff, Cl,

$$-CO{-}({-}OC_2H_4{-})_x{-}OSO_3X,$$

-COOX oder -$SO_3X$ bedeutet,
$R_2$ Wasserstoff, $C_1$-$C_8$-Alkyl, $C_7$-$C_9$-Phenylalkyl oder eine Gruppe -Y-Z bedeutet,
$R_3$ Wasserstoff oder eine Gruppe -Y-Z bedeutet, X Li, Na, K oder

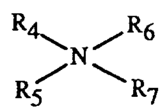

ist, x eine ganze Zahl von 2 bis 8 ist,
Y eine Gruppe -$R_8$- oder -$R_8$-COO-$R_9$- bedeutet, worin $R_8$ und $R_9$ $C_1$-$C_4$-Alkylen bedeuten, und
Z eine Gruppe -COOX oder -$SO_3X$ bedeutet, wobei in den Verbindungen der Formel I' mindestens zwei Gruppen -$SO_3X$ enthalten sind.

**13.** Verbindungen gemäss Anspruch 10 der Formel I', worin $R_2$ in Orthostellung und $R_3$ in Parastellung zur OH-Gruppe stehen.

**14.** Verwendung von Verbindungen der Formel I gemäss Definition in Anspruch 1 als Lichtschutzmittel für wässrige Tinten.

**15.** Aufzeichnungsmaterial für das Tintenstrahldruckverfahren, enthaltend mindestens eine Verbindung der Formel I.

## Claims

**1.** An aqueous ink comprising as light stabiliser at least one compound of formula I

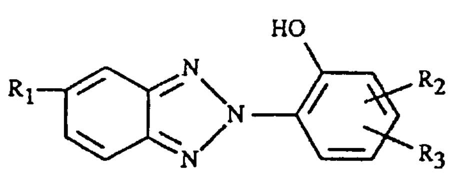

I

wherein $R_1$ is hydrogen, halogen, $C_1$-$C_4$alkyl, $C_1$-$C_4$alkoxy, -COO-$R_9$-$OSO_3X$,-COOX or -$SO_3X$,
$R_2$ is hydrogen, OH, $C_1$-$C_8$alkyl, $C_5$-$C_6$cycloalkyl, phenyl, naphthyl, $C_7$-$C_{12}$aralkyl, halogen, $C_1$-$C_8$alkoxy or a group -Y-Z,
$R_3$ is hydrogen, $C_1$-$C_8$alkyl, a group -Y-Z or a group -Y-O-Z',
X is hydrogen or M,
M is Li, Na, K, $\frac{1}{2}$ Mg, $\frac{1}{2}$ Ca, $\frac{1}{2}$ Zn, $\frac{1}{2}$ Co (II), $\frac{1}{2}$ Cu (II), $\frac{1}{2}$ Ni (II), $\frac{1}{3}$ Cr (III), $\frac{1}{3}$ Fe (III),

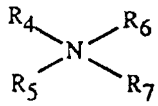, 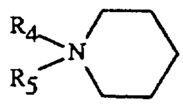, 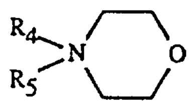 or ½ 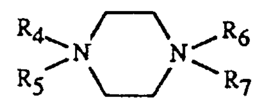

wherein $R_4$, $R_5$, $R_6$ and $R_7$ are each independently of one another hydrogen, $C_1$-$C_{12}$alkyl, hydroxyethyl, cyclohexyl, phenyl or benzyl,
Y is a direct bond or a divalent group of one of the following formulae:
-$R_8$-, -$R_8$-CONH-$R_9$-,

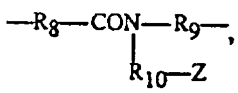, 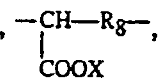, 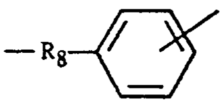,

-$R_8$-COO-$R_9$-,
-$R_8$-$SO_2$-$R_9$-, -$R_8$-$SO_2$NH-$R_9$-,,

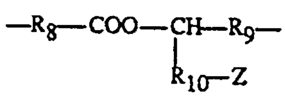 or 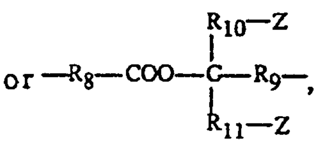,

wherein $R_8$, $R_9$, $R_{10}$ and $R_{11}$ are each independently of one another $C_1$-$C_{20}$alkylene which may be substituted by 1-10 hydroxyl groups or are $C_2$-$C_{20}$alkylene which is interrupted by 1-10 -O- or -NH-, and
Z is a group -COOX or -$SO_3$X, and Z' is -$SO_3$X,
the compound of formula I containing at least one group of formula -COOX or -$SO_3$X.

2. An ink according to claim 1, comprising a compound of formula I wherein $R_1$ is hydrogen, halogen, $C_1$-$C_4$alkyl, $C_1$-$C_4$alkoxy, -COO-$R_9$-$OSO_3$X, - COOX or -$SO_3$X,
$R_2$ is hydrogen, $C_1$-$C_8$alkyl, $C_1$-$C_9$phenylalkyl or a group -Y-Z,
$R_3$ is hydrogen, $C_1$-$C_4$alkyl, a group -Y-Z or a group -Y-O-Z',
X is hydrogen or M,
M is Li, Na, K,

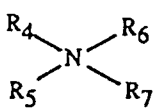, 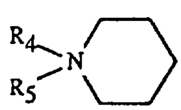 or 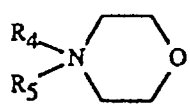,

wherein $R_4$, $R_5$, $R_6$ and $R_7$ are each independently of one another H, $C_1$-$C_4$alkyl or hydroxyethyl,
Y is a direct bond or one of the groups -$R_8$-, -$R_8$-CONH-$R_9$-,

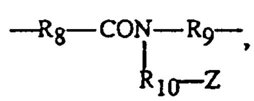,

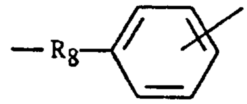,

$-R_8-COO-R_9-$ or

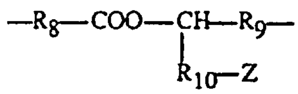,

wherein $R_8$ is $C_1$-$C_6$alkylene and $R_9$ and $R_{10}$ are each independently of the other $C_1$-$C_{10}$alkylene which may be substituted by 1 or 2 hydroxyl groups or are $C_2$-$C_{20}$alkylene which is interrupted by 1-10 -O-, and Z is a group -COOX or $-SO_3X$ and Z' is $-SO_3X$.

**3.** An ink according to claim 2, comprising a compound of formula I wherein $R_1$ is hydrogen, chloro,

$$-CO-(-OC_2H_4-)_x-OSO_3X,$$

-COOX or $-SO_3X$,
$R_2$ is hydrogen, $C_1$-$C_8$alkyl, $C_7$-$C_9$phenylalkyl or a group -Y-Z,
$R_3$ is hydrogen or a group -Y-Z and x is an integer from 2 to 8, and M, X, Y and Z are as defined in claim 2.

**4.** An ink according to claim 3, comprising a compound of formula I wherein $R_1$, $R_2$ and $R_3$ are as defined in claim 3, X is Li, Na, K or

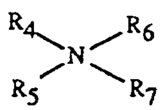,

Y is a direct bond or one of the groups $-R_8-$, $-R_8-CONH-R_9-$,

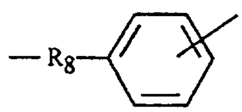

or $-R_8-COO-R_9-$,
Z is a group -COOX or $-SO_3X$, and $R_4$, $R_5$, $R_6$, $R_7$, $R_8$ and $R_9$ are as defined in claim 2.

**5.** An ink according to claim 1, comprising a compound of formula I wherein $R_2$ is in ortho-position and $R_3$ is in para-position to the OH group.

**6.** An ink according to claim 1, which contains 0.01 - 20% by weight of at least one compound of formula I.

**7.** An ink according to claim 1, which contains 0.1 - 10% by weight of at least one compound of formula I.

**8.** An ink according to claim 1, which contains at least 30% by weight of water.

**9.** The use of an ink according to claim 1 for ink jet printing.

**10.** A compound of formula I'

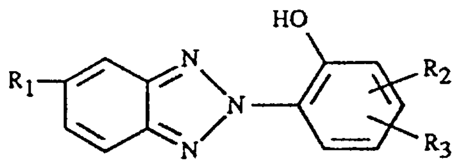

I'

wherein $R_1$ is hydrogen, halogen, $C_1$-$C_4$alkyl, $C_1$-$C_4$alkoxy, -COO-$R_9$-$OSO_3X$,-COOM or -$SO_3X$, $R_2$ is hydrogen, OH, $C_1$-$C_8$alkyl, $C_5$-$C_6$cycloalkyl, phenyl, naphthyl, $C_7$-$C_{12}$aralkyl, halogen, $C_1$-$C_8$alkoxy or a group -Y-Z,
$R_3$ is hydrogen, $C_1$-$C_8$alkyl or a group -Y-Z,
X is hydrogen or M,
M is Li, Na, K, $\frac{1}{2}$ Mg, $\frac{1}{2}$ Ca, $\frac{1}{2}$ Zn, $\frac{1}{2}$ Co (II), $\frac{1}{2}$ Cu (II), $\frac{1}{2}$ Ni (II), $\frac{1}{3}$ Cr (III), $\frac{1}{3}$ Fe (III),

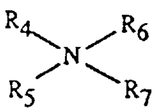 , 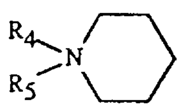 , 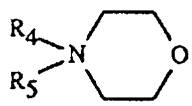 or $\frac{1}{2}$ 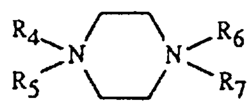 ,

wherein $R_4$, $R_5$, $R_6$ and $R_7$ are each independently of one another hydrogen, $C_1$-$C_{12}$alkyl, hydroxyethyl, cyclohexyl, phenyl or benzyl,
Y is one of the groups -$R_8$-, -$R_8$-CONH-$R_9$-,

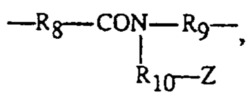 , 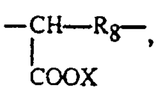 ,

-$R_8$-COO-$R_9$-,
-$R_8$-$SO_2$-$R_9$-, -$R_8$-$SO_2$NH-$R_9$-,

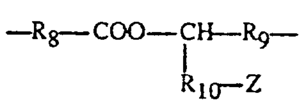 or 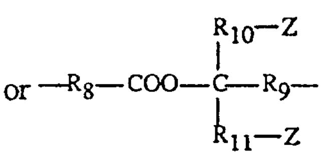 ,

wherein $R_8$, $R_9$, $R_{10}$ and $R_{11}$ are each independently of one another $C_1$-$C_{20}$alkylene which may be substituted by 1-10 hydroxyl groups or are $C_2$-$C_{20}$alkylene which is interrupted by 1-10 -O- or -NH-, and Z is a group -COOM or -$SO_3X$, the compound of formula I' containing at least one group -COOX and/or at least one group -$SO_3X$.

**11.** A compound according to claim 9, which contains two groups -$SO_3X$.

**12.** A compound according to claim 11, wherein $R_1$ is hydrogen, Cl,

$$-CO{+}OC_2H_4{+}_x OSO_3X,$$

-COOX or -$SO_3X$,
$R_2$ is hydrogen, $C_1$-$C_8$alkyl, $C_7$-$C_9$phenylalkyl or a group -Y-Z,
$R_3$ is hydrogen or a group -Y-Z,
X is Li, Na, K or

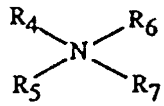 ,

x is an integer from 2 to 8,
Y is a group $-R_8-$ or $-R_8-COO-R_9-$, wherein $R_8$ and $R_9$ are $C_1-C_4$alkylene, and
Z is a group -COOX or $-SO_3X$, the compound of formula I' containing at least two groups $-SO_3X$.

**13.** A compound according to claim 10 of formula I', wherein $R_2$ is in ortho-position and $R_3$ is in para-position to the OH group.

**14.** The use of a compound of formula I as defined in claim 1 as a light stabiliser for aqueous inks.

**15.** A recording material for the ink jet printing process, comprising at least one compound of formula I.

**Revendications**

**1.** Encre à l'eau, contenant comme agent de protection contre la lumière au moins un composé de formule (I)

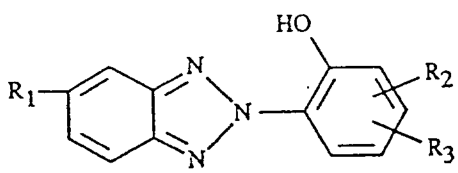 I

où $R_1$ est un hydrogène, un halogène ou un radical alkyle en $C_1-C_4$, alcoxy en $C_1-C_4$, $-COO-R_9-OSO_3X$, -COOX ou $-SO_3X$,

$R_2$ est un hydrogène, OH ou un radical alkyle en $C_1-C_8$, cycloalkyle en $C_5-C_6$, phényle, naphtyle, aralkyle en $C_7-C_{12}$, halogéno, alcoxy en $C_1-C_8$ ou encore un groupe -Y-Z,

$R_3$ est un hydrogène ou un radical alkyle en $C_1-C_8$, un groupe -Y-Z ou un groupe -Y-O-Z',

X est un hydrogène ou représente M,

M est Li, Na, K, $\frac{1}{2}$ Mg, $\frac{1}{2}$ Ca, $\frac{1}{2}$ Zn, $\frac{1}{2}$ Co(III), $\frac{1}{2}$ Cu(II), $\frac{1}{2}$ Ni(II), 1/3 Cr(III), 1/3 Fe(III)

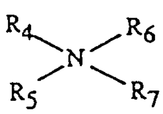 , 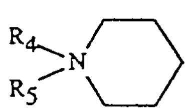 , 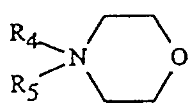 ou $\frac{1}{2}$ 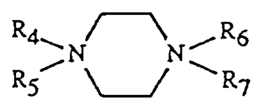

où $R_4$, $R_5$, $R_6$ et $R_7$, indépendamment les uns des autres, sont chacun un hydrogène ou un radical alkyle en $C_1-C_{12}$,hydroxyéthyle, cyclohexyle, phényle ou benzyle,

Y est une liaison directe ou un groupe divalent ayant l'une des formules suivantes :
$-R_8-$, $-R_8-CONH-R_9-$,

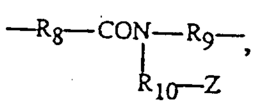 , 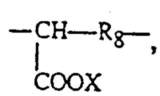 , 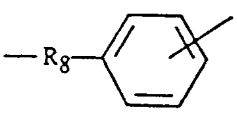 ,

$-R_8-COO-R_9-$, $-R_8-SO_2-R_9-$, $-R_8-SO_2NH-R_9-$,,

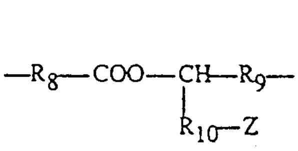 ou 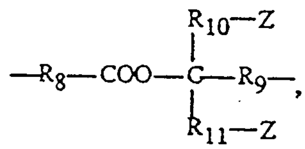,

où $R_8$, $R_9$, $R_{10}$ et $R_{11}$, indépendamment les uns des autres, sont chacun un radical alkylène en $C_1$-$C_{20}$, pouvant être substitué par 1 à 10 groupes hydroxyle, ou encore un radical alkylène en $C_2$-$C_{20}$ interrompu par 1 à 10 atomes -O- ou groupements -NH-, et

Z est un groupe -COOX ou -$SO_3$X, Z' est -$SO_3$X,

le composé de formule (I) contenant au moins un groupe de formule -COOX ou -$SO_3$X.

**2.** Encre selon la revendication 1, qui contient un composé de formule (I) où $R_1$ est un hydrogène, un halogène, ou un radical alkyle en $C_1$-$C_4$, alcoxy en $C_1$-$C_4$, -COO-$R_9$-O$SO_3$X, -COOX ou -$SO_3$X,

$R_2$ est un hydrogène ou un radical alkyle en $C_1$-$C_8$, phénylalkyle en $C_7$-$C_9$ ou encore un groupe -Y-Z-,

$R_3$ est un hydrogène ou un radical alkyle en $C_1$-$C_4$, un groupe -Y-Z ou un groupe -Y-O-Z',

X est un hydrogène ou M,

M est Li, Na, K,

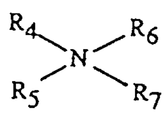, 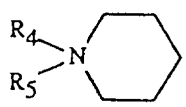 ou 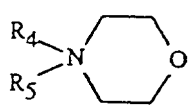

où $R_4$, $R_5$, $R_6$ et $R_7$, indépendamment les uns des autres, sont chacun H ou un radical alkyle en $C_1$-$C_4$ ou hydroxyéthyle,

Y est une liaison directe ou l'un des groupes -$R_8$-, -R-8-CONH-$R_9$-,

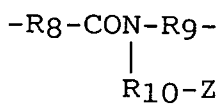

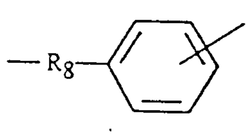,

-$R_8$-COO-$R_9$- ou

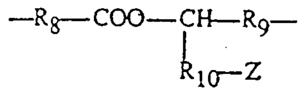

où $R_8$ est un radical alkylène en $C_1$-$C_6$, et $R_9$ et $R_{10}$, indépendamment l'un de l'autre, sont chacun un radical alkylène en $C_1$-$C_{10}$, pouvant être substitué par un ou deux groupes hydroxyle, ou encore un radical alkylène en $C_2$-$C_{20}$ interrompu par 1 à 10 atomes -O-, et

Z est un groupe -COOX ou -$SO_3$X, et Z' est -$SO_3$X.

**3.** Encre selon la revendication 2, qui contient un composé de formule (I), où $R_1$ est un hydrogène, le chlore, $-CO-(OC_2H_4)_x-OSO_3X$, -COOX ou $-SO_3X$,

$R_2$ est un hydrogène ou un radical alkyle en $C_1$-$C_8$, phénylalkyle en $C_7$-$C_9$ ou un groupe -Y-Z-,

$R_3$ est un hydrogène ou un groupe -Y-Z, et x est un nombre entier de 2 à 8.

**4.** Encre selon la revendication 3, qui contient un composé de formule (I) dans laquelle $R_1$, $R_2$ et $R_3$ ont les significations données dans la revendication 3, X est Li, Na, K ou

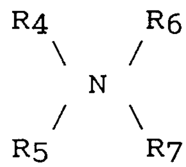

Y est une liaison directe ou l'un des groupes $-R_8-$, $-R_8-CONH-R_9$,

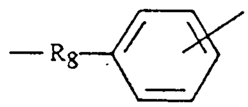

ou encore $-R_8-COO-R_9-$,

Z est un groupe -COOX ou $-SO_3X$, et $R_4$, $R_5$, $R_6$, $R_7$, $R_8$ et $R_9$ ont les significations données dans la revendication 2.

**5.** Encre selon la revendication 1, qui contient un composé de formule (I) dans laquelle $R_2$ est en position ortho et $R_3$ en position para par rapport au groupe OH.

**6.** Encre selon la revendication 1, qui contient de 0,01 à 20 % en poids d'au moins un composé de formule (I).

**7.** Encre selon la revendication 1, qui contient de 0,1 à 10 % en poids d'au moins un composé de formule (I).

**8.** Encre selon la revendication 1, qui contient au moins 30 % en poids d'eau.

**9.** Utilisation d'une encre selon la revendication 1 pour l'impression au jet d'encre.

**10.** Composés de formule (I')

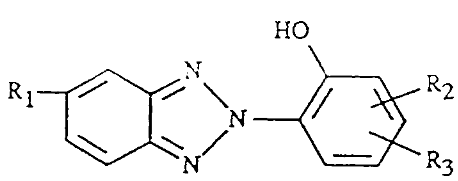

I'

où $R_1$ est un hydrogène, un halogène ou un radical alkyle en $C_1$-$C_4$, alcoxy en $C_1$-$C_4$, $-COO-R_9-OSO_3X$, -COOM ou $-SO_3X$,

$R_2$ est un hydrogène, OH ou un radical alkyle en $C_1$-$C_8$, cycloalkyle en $C_5$-$C_6$, phényle, naphtyle, aralkyle en $C_7$-$C_{12}$, halogéno, alcoxy en $C_1$-$C_8$ ou encore un groupe -Y-Z,

$R_3$ est un hydrogène ou un radical alkyle en $C_1$-$C_8$, un groupe -Y-Z,

X est un hydrogène ou représente M,

M est Li, Na, K, $\frac{1}{2}$ Mg, $\frac{1}{2}$ Ca, $\frac{1}{2}$ Zn, $\frac{1}{2}$ Co(III), $\frac{1}{2}$ Cu(II), $\frac{1}{2}$ Ni(II), 1/3 Cr(III), 1/3 Fe(III)

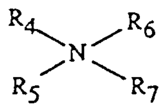, 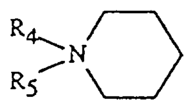, 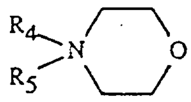 ou ½ 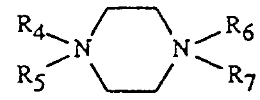

où $R_4$, $R_5$, $R_6$ et $R_7$, indépendamment les uns des autres, sont chacun un hydrogène ou un radical alkyle en $C_1$-$C_{12}$,hydroxyéthyle, cyclohexyle, phényle ou benzyle,

X est un hydrogène ou M,

Y est une liaison directe ou un groupe divalent ayant l'une des formules suivantes : $-R_8-$, $-R_8-CONH-R_9-$,

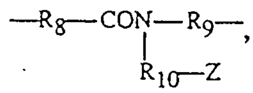, 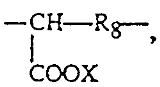,

$-R_8-COO-R_9-$, $-R_8-SO_2-R_9-$, $-R_8-SO_2NH-R_9-$,

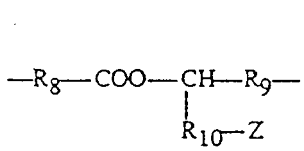 ou 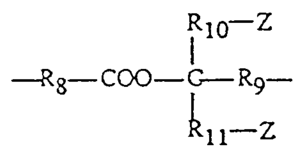

où $R_8$, $R_9$, $R_{10}$ et $R_{11}$, indépendamment les uns des autres, sont chacun un radical alkylène en $C_1$-$C_{20}$, pouvant être substitué par 1 à 10 groupes hydroxyle, ou encore un radical alkylène en $C_2$-$C_{20}$ interrompu par 1 à 10 atomes -O- ou groupements -NH-, et

Z est un groupe -COOM ou $-SO_3X$, Z' est $-SO_3X$,

le composé de formule (I') contenant au moins un groupe de formule -COOX ou $-SO_3X$.

**11.** Composés selon la revendication 9, qui contiennent deux groupes $-SO_3X$.

**12.** Composés selon la revendication 11, où $R_1$ est un hydrogène, le chlore, $-CO-(OC_2H_4)_x-OSO_3X$, -COOX ou $-SO_3X$,

$R_2$ est un hydrogène ou un radical alkyle en $C_1$-$C_8$, phénylalkyle en $C_7$-$C_9$ ou un groupe -Y-Z-,

$R_3$ est un hydrogène ou un groupe -Y-Z,

X est Li, Na, K ou

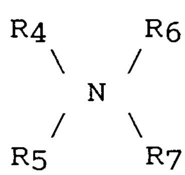

x est un nombre entier de 2 à 8,

Y est un groupe $-R_8-$ ou $-R_8-COO-R_9-$, où $R_8$ et $R_9$ sont chacun un radical alkylène en $C_1$-$C_4$,

et Z est un groupe -COOX ou $-SO_3X$, les composés de formule (I') contenant au moins deux groupes $-SO_3X$.

**13.** Composés selon la revendication 10 de formule I', dans laquelle $R_2$ est en position ortho et $R_3$ en position para par rapport au groupe OH.

**14.** Utilisation de composés de formule (I) selon la définition de la revendication 1 en tant qu'agent de protection de la lumière pour encres à l'eau.

**15.** Matériau d'enregistrement pour le procédé d'impression au jet d'encre, qui contient au moins un composé de formule (I).